# EUROPEAN PATENT SPECIFICATION

(11) **EP 3 587 553 B1**
(45) Date of publication and mention of the grant of the patent: **23.08.2023**
(21) Application number: 19181998.6
(22) Date of filing: 24.06.2019
(51) Int. Cl.: C12M 1/34

(54) **CELL OBSERVATION APPARATUS**
ZELLENBEOBACHTUNGSVORRICHTUNG
APPAREIL D'OBSERVATION DE CELLULES

(30) Priority: 29.06.2018 JP 2018125387
(43) Date of publication of application: 01.01.2020
(73) Proprietor: Shibuya Corporation, Kanazawa-shi, Ishikawa 920-8681 (JP)
(72) Inventor: Kesyou, Toru, Kanazawa-shi, Ishikawa 920-8681 (JP); Watanabe, Daisuke, Kanazawa-shi, Ishikawa 920-8681 (JP); Konishi, Tomohiro, Kanazawa-shi, Ishikawa 920-8681 (JP)
(74) Representative: Mewburn Ellis LLP

(56) References cited:
- EP-A1- 3 211 469
- JP-A- H10 332 792
- US-A1- 2004 256 571

## Description

### BACKGROUND OF THE INVENTION

### Field of the Invention

The present invention relates to a cell observation apparatus, and more particularly to a cell observation apparatus comprising a camera imaging cells contained in a culture vessel and a lighting irradiating the cells with inspection light.

### Description of the Related Art

There has conventionally been known a cell observation apparatus observing a culture state of cells in culture using a camera. At this time, in order to clearly obtain an image of the cells, the cells have been irradiated with inspection light. Various approaches have been tried with respect to an irradiation direction of the inspection light relative to an imaging direction of the camera at this time.

For example, there has been known a cell observation apparatus in which a lighting is provided on a side of a culture vessel, inspection light is made to be incident from below on a liquid surface of a culture fluid contained in the culture vessel, and this is imaged by a camera provided below the culture vessel (International Publication No. WO2015/174356). This cell observation apparatus can handle culture vessels stacked in multiple stages.

Regarding the multi-stage culture vessel, there has also been known a cell observation apparatus in which a camera and a lighting are provided coaxially facing each other, and the culture vessel is positioned between the camera and the lighting (Japanese Patent No. 4049263).

Here, the cells and tissues for use in treating regenerative medicine and the like have come to be handled in a sterile environment. Thus, it is desirable that the operation of observing the cells in the culture vessel is also performed in a sterile chamber where the sterile environment is maintained.

However, in order to maintain the sterile condition of the sterile chamber, the sterile chamber needs to be periodically decontaminated by a decontamination medium such as hydrogen peroxide vapor. For this reason, an optical system such as the lighting and the camera constituting the observation apparatus needs to be configured to be protected from heat and decontamination media, but the configurations disclosed in International Publication No. WO2015/174356 and Japanese Patent No. 4049263 lack the consideration of the optical system used in the sterile chamber. EP 3 211 469 discloses an apparatus for sample observation characterized by an observation container with a camera and light sources both beneath a single plane of observation, where the sample is positioned, with fixed positions of the light sources laterally to the camera, without adjusting mechanisms for light and camera and no spotlight sources.

In addition, a multi-stage culture vessel requires the camera and the lighting to be moved along a height direction of the vessel to observe the cells in each stage, which involves a problem that the apparatus becomes more complicated and larger, and it is more infeasible to contain the culture vessel in the sterile chamber.

In view of the above problem, the present invention has been made, and an object of the present invention is to provide a cell observation apparatus capable of providing a camera and a lighting outside a sterile chamber and capable of clearly imaging cells.

### SUMMARY OF THE INVENTION

As described above, the cell observation apparatus according to the invention of claim 1 is a cell observation apparatus comprising a camera imaging cells contained in a culture vessel and a lighting irradiating the cells with inspection light, characterized by comprising:
a sterile chamber, an inside of which is maintained in a sterile condition and which contains the culture vessel; and a transparent observation window provided in a bottom portion of the sterile chamber and having the culture vessel placed thereabove, wherein
the camera and the lighting are provided below the observation window, and the cells in the culture vessel are imaged through the observation window by setting the focus of the camera to a bottom surface of the culture vessel,
the lighting emits the inspection light as spot light to the bottom surface of the culture vessel by emitting light in a shape of a spot where the light is not diffused, wherein an imaging range of the camera is positioned inside an irradiation range of the inspection light on the bottom surface of the culture vessel, and
the cell observation apparatus is provided with an angle adjustment mechanism such that an imaging direction of the camera and an irradiation direction of the lighting are set so as not to be at the same angle relative to an axis orthogonal to the bottom surface of the culture vessel.

The present invention can configure a cell observation apparatus capable of providing the camera and the lighting outside the sterile chamber and capable of clearly imaging cells.

### BRIEF DESCRIPTION OF THE DRAWING

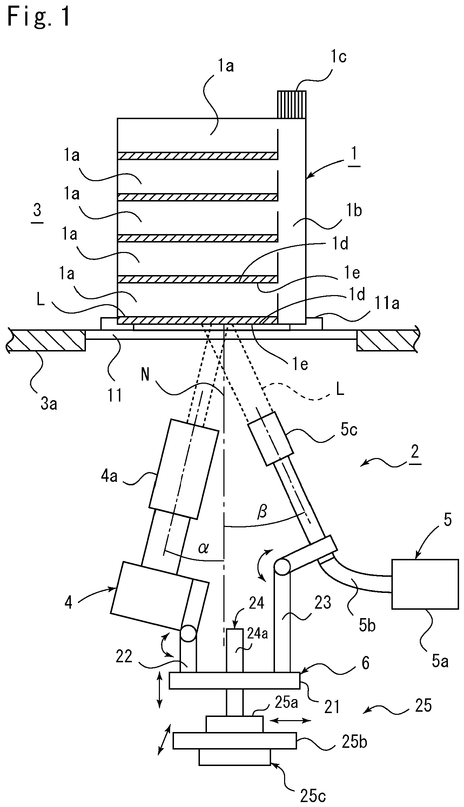

FIG. 1 is a side view of an observation apparatus according to the present embodiment.

### DETAILED DESCRIPTION OF THE PREFERRED EMBODIMENTS

Hereinafter, an illustrated embodiment will be described. FIG. 1 is a configuration view of a cell observation apparatus 2 observing cells cultured inside a culture vessel 1.

The cell observation apparatus 2 comprises: a sterile chamber 3, an inside of which is maintained in a sterile condition and which contains the culture vessel 1; a camera 4 provided below the sterile chamber 3; a lighting 5 provided also below the sterile chamber 3; and a moving unit 6 moving the camera 4 and lighting 5.

The culture vessel 1 is a box-shaped container made of a transparent material and includes five stages of container portions 1a thereinside. The cells are contained in each stage of the container portions 1a in a state of being immersed in a culture fluid L.

A vertically penetrating passage 1b is formed at a position adjacent to each of the container portions 1a in the culture vessel 1. An openable and closable cap 1c is provided on an upper portion of the passage 1b.

The cells and the culture fluid to be observed in the present embodiment are transparent, and the cells are cultured in a state of being thinly adhered to a bottom surface 1d of each container portion 1a.

The sterile chamber 3 is configured, for example, as an isolator or a culture incubator that handles the cells, the inside of which is maintained in a sterile condition. Further, the inside of the sterile chamber 3 can be periodically decontaminated by a decontamination medium such as hydrogen peroxide vapor.

A colorless transparent observation window 11 made of glass, polycarbonate, or the like is formed in at least a part of a bottom portion 3a of the sterile chamber 3, and the observation window 11 is horizontally provided.

In the present embodiment, the cells contained in the culture vessel 1 can be observed from outside the sterile chamber 3 in a state where the culture vessel 1 is placed on a positioning support member 11a provided on the upper surface of the observation window 11.

The operation of placing the culture vessel 1 on the observation window 11 may be performed manually using an unillustrated glove provided in the sterile chamber 3 or may be performed automatically using a robot or other transport means.

The camera 4 is provided below the observation window 11 and is oriented toward a lower surface 1e of the culture vessel 1 so as to image the cells inside the culture vessel 1 through the observation window 11.

Specifically, in order to image the cells adhered to a bottom surface 1d of the lowermost container portion 1a, the focus is set to the bottom surface 1d of the container portion 1a.

Further, the camera 4 has a high-resolution short focus lens 4a. For this reason, the imaging range of the camera 4 is an extremely narrow range, for example, a range of 2.1 mm × 1.7 mm relative to the bottom surface 1d of the container portion 1a.

Further, when an imaging angle α of the camera 4 is greatly inclined relative to the lower surface 1e of the culture vessel 1, the range of focus becomes narrow in the imaging range. When the imaging angle α is a right angle, namely, approaches 0°, it is difficult to receive reflected light from the transparent cells adhered to the bottom surface 1d of the container portion 1a. Therefore, the imaging angle α of the camera 4 is set in a range of 10 to 30°, preferably around 20° relative to a virtual axis N (normal line to the lower surface 1e) orthogonal to the lower surface 1e of the culture vessel 1.

The lighting 5 includes an LED spot lighting and comprises a main body portion 5a, a flexible arm portion 5b extending from the main body portion 5a, and an irradiation portion 5c emitting the inspection light L toward the lower surface 1e of the culture vessel 1.

An irradiation angle β of the inspection light L emitted by the irradiation portion 5c is set to be inclined relative to the virtual axis N orthogonal to the lower surface 1e of the culture vessel 1 and an irradiation end of the lighting 5 and a light-receiving end of the camera 4 are provided to face each other across the virtual axis N.

Further, the lighting 5 emits the inspection light L in a spot shape in which the light is not diffused and emits the inspection light L in a narrow range forming a part of the bottom surface 1d of the container portion 1a, so that for example, a circular range of about 6 mm in diameter becomes the irradiation range of the inspection light L.

At this time, the imaging direction of the camera 4 and the irradiation direction of the lighting 5 are set so that the imaging range by the camera 4 is positioned inside the irradiation range of the inspection light L, thereby allowing the camera 4 to image the cells irradiated with the inspection light L.

Here, the inspection light L emitted by the lighting 5 is reflected mainly at a boundary portion between the transparent culture fluid and the cells inside the container portion 1a and is received by the camera 4. By using spot light so that the inspection light L is not diffused, the direction of the inspection light L emitted to the cells can be aligned and the direction of the reflected light received by the camera 4 can also be aligned, thereby to increase the difference between light and dark, which can highlight the boundary between the culture fluid and the cells, that is, the contour of the cells.

On the other hand, when the inspection light L is emitted in a diffuse manner, the inspection light L is reflected in various directions in the culture fluid, and the reflected light is incident on the camera 4 from various directions. As a result, the above described difference in light and dark cannot be obtained, the entire image becomes bright, and the contour of the cells becomes unclear.

When the irradiation angle β of the lighting 5 is greatly inclined relative to the lower surface 1e of the culture vessel 1, the spot diameter of the inspection light L increases and the inspection light L is incident while being diffused. In addition, when the irradiation angle β approaches a right angle, namely, 0°, the amount of light that escapes above the transparent cells increases and the amount of reflected light decreases. Therefore, the irradiation angle β of the lighting 5 is set in a range of 0 to 40° relative to the virtual axis N, more preferably in a range of 10 to 30°.

Further, in the present embodiment, the imaging angle α of the camera 4 and the irradiation angle β of the lighting 5 are set to be different relative to the virtual axis N orthogonal to the lower surface 1e of the culture vessel.

The reason for this is that if the imaging angle α of the camera 4 and the irradiation angle β of the lighting 5 are set to be the same, the angle of incidence and the angle of reflection become the same relative to the normal line (virtual axis N) of the lower surface 1e of the culture vessel 1 as the boundary surface, and the reflected light reflected from the lower surface 1e is received directly by the camera 4, causing the entire image to be all white, known as blown out highlights or clipped whites. As a result, the cells cannot be imaged at all.

In light of this, the imaging direction of the camera 4 and the irradiation direction of the lighting 5 are set so as not to be the same angle, and thereby the imaging angle α of the camera 4 is made different from the irradiation angle β of the lighting 5, thereby to prevent the camera 4 from directly receiving the reflected light reflected from the lower surface 1e of the culture vessel 1.

Specifically, when the imaging angle α of the camera 4 is set to 20° relative to the virtual axis N, the irradiation angle β of the lighting 5 is set in a range of 0 to 40° other than 20° relative to the virtual axis N.

The moving unit 6 is configured to move the camera 4 and the lighting 5 integrally in an up/down direction and a lateral direction while maintaining that the imaging direction by the imaging angle α of the camera 4 and the irradiation direction by the irradiation angle β of the lighting 5 are not changed.

Specifically, the camera 4 and the lighting 5 are fixed to a moving table 21 via the angle adjustment mechanisms 22 and 23, respectively. As described above, by the angle adjustment mechanisms 22 and 23, the imaging angle α of the camera 4 and the irradiation angle β of the lighting 5 can be adjusted.

The moving table 21 is provided so as to be moved upward and downward by a lifting/lowering unit 24 and to be moved laterally by a lateral moving unit 25.

The lifting/lowering unit 24 includes an up/down drive device 24a for moving the moving table 21 in an up/down direction.

The lateral moving unit 25 includes a left/right drive device 25b that reciprocates a left/right moving table 25a supporting the up/down drive device 24a in a left/right direction on the drawing, and a front/back drive device 25c that reciprocates the left/right drive device 25b in a front/back direction on the drawing.

As described above, the imaging range by the camera 4 is extremely narrow and is a part of the bottom surface 1d of the container portion 1a of the culture vessel 1. Thus, in order to observe the cells in the culture vessel 1 more accurately, a plurality of portions of the bottom surface 1d need to be imaged.

In light of this, a plurality of portions of the bottom surface 1d of the container portion 1a can be imaged by causing the lateral moving unit 25 of the moving unit 6 to laterally move the camera 4 and the lighting 5 integrally while maintaining the imaging direction and the irradiation direction of the inspection light L.

Further, the present embodiment can observe the cells contained not only in the lowermost container portion 1a but also in the second-from-lowermost container portion 1a in the multi-stage culture vessel 1 by causing the moving unit 6 to move the camera 4 and the lighting 5 integrally in the up/down direction while maintaining the imaging direction and the irradiation direction of the inspection light L.

More specifically, the focus of the camera 4 can be changed to be set to the bottom surface 1d of the second-from-lowermost container portion 1a from a state of being set to the bottom surface 1d of the lowermost container portion 1a by causing the lifting/lowering unit 24 of the moving unit 6 to move the camera 4 and the lighting 5 integrally upward while maintaining the imaging direction and the irradiation direction of the inspection light L.

Further, the inspection light L emitted by the lighting 5 passes through the lowermost container portion 1a and is emitted in a spot shape to the cells adhered to the bottom surface 1d of the container portion 1a of the second-from-lowermost container portion 1a. Then, the camera 4 images the cells inside the irradiation range as the imaging range.

The second-from-lowermost image was not as sharp as the lowermost image, but was sufficient to confirm the condition of cells (for example, the degree of proliferation).

Note that by the configuration of the present embodiment, the cells in the third-from-lowermost container portion 1a was not imaged with sufficient clarity for observation, but it can be considered that the cells in a further upper stacked container portion 1a can also be observed by adjusting the resolution of the camera 4 and the intensity of the inspection light L of the lighting 5.

However, in the observation of the cells in the multi-stage culture vessel 1, all the container portions 1a in all stages are not necessarily imaged. In order to confirm the degree of cell proliferation as in the present embodiment, it is sufficient to observe the lowermost container portion and the second-from-lowermost container portion.

Note that another embodiment may be configured such that the moving unit 6 is provided inside the sterile chamber 3, the camera 4 and the lighting 5 are fixed, and the culture vessel 1 is held and moved. Note also that still another embodiment may be configured such that the camera 4 and the lighting 5 are moved mutually relative to the culture vessel 1. In this case, it may be configured that the camera 4 and the lighting 5 are moved relatively with respect to the culture vessel 1 (for the up/down movement and lateral movement).

It should be noted that the multi-stage culture vessel 1 is not limited to the configuration in which a plurality of container portions 1a are stacked in multiple stages thereinside as in the embodiment, but it may be configured that a plurality of single-stage culture vessels are stacked, which enables the same observation as in the embodiment.

### [Reference Signs List]

- 1: culture vessel
- 2: observation apparatus
- 3: sterile chamber
- 4: camera
- 5: lighting
- 11: observation window
- L: inspection light
- α: imaging angle of camera
- β: irradiation angle of lighting

## Claims

1. A cell observation apparatus (2) comprising a camera (4) imaging cells contained in a culture vessel (1) and a lighting (5) irradiating the cells with inspection light (L), **characterized by** comprising:
a sterile chamber (3), an inside of which is maintained in a sterile condition and which contains the culture vessel (1); and a transparent observation window (11) provided in a bottom portion of the sterile chamber (3) and having the culture vessel placed thereabove, wherein
the camera (4) and the lighting (5) are provided below the observation window, and the cells in the culture vessel (1) are imaged through the observation window (11) by setting the focus of the camera to a bottom surface of the culture vessel,
the lighting (5) emits the inspection light (L) as spot light to the bottom surface of the culture vessel (1) by emitting light in a shape of a spot where the light is not diffused, wherein an imaging range of the camera (4) is positioned inside an irradiation range of the inspection light (L) on the bottom surface of the culture vessel, and
the cell observation apparatus (2) is provided with an angle adjustment mechanism such that an imaging direction of the camera (4) and an irradiation direction of the lighting (L) are set so as not to be at the same angle relative to an axis orthogonal to the bottom surface of the culture vessel (1).

2. The cell observation apparatus (2) according to claim 1, **characterized by** comprising
a moving unit (6) moving the camera (4) and the lighting (5) integrally while maintaining the imaging direction of the camera and the irradiation direction of the lighting, wherein
the moving unit (6) sets the focus of the camera to the bottom surface of each stage of the stacked culture vessels by moving the camera (4) and the lighting (5) upward and downward.

3. The cell observation apparatus (2) according to any one of claims 1 and 2, **characterized by** comprising
a moving unit (6) moving the camera (4) and the lighting (5) integrally while maintaining the imaging direction of the camera and the irradiation direction of the lighting, wherein
the moving unit (6) changes the irradiation range of the inspection light (L) and the imaging range of the camera (4) on the bottom surface of the culture vessel (1) by laterally moving the camera (4) and the lighting (5) .

## Patentansprüche

1. Zellbeobachtungsvorrichtung (2), die eine Kamera (4), die in einem Kulturgefäß (1) enthaltene Zellen abbildet, und eine Beleuchtung (5) umfasst, die die Zellen mit Inspektionslicht (L) bestrahlt, **dadurch gekennzeichnet, dass** sie Folgendes umfasst:
eine sterile Kammer (3), deren Inneres in einem sterilen Zustand gehalten wird und die das Kulturgefäß (1) enthält; und ein transparentes Beobachtungsfenster (11), das in einem Bodenabschnitt der sterilen Kammer (3) bereitgestellt und das Kulturgefäß darauf platziert ist, wobei
die Kamera (4) und die Beleuchtung (5) unter dem Beobachtungsfenster bereitgestellt sind, und die Zellen in dem Kulturgefäß (1) durch das Beobachtungsfenster (11) abgebildet werden, indem der Fokus der Kamera auf eine Bodenfläche des Kulturgefäßes eingestellt wird,
die Beleuchtung (5) das Inspektionslicht (L) als Lichtfleck auf die Bodenfläche des Kulturgefäßes (1) ausstrahlt, indem Licht in Form eines Flecks ausgestrahlt wird, bei dem das Licht nicht gestreut wird, wobei ein Abbildungsbereich der Kamera (4) innerhalb eines Bestrahlungsbereichs des Inspektionslichts (L) auf der Bodenfläche des Kulturgefäßes positioniert ist, und
die Zellbeobachtungsvorrichtung (2) mit einem Winkeleinstellmechanismus bereitgestellt ist, sodass eine Abbildungsrichtung der Kamera (4) und eine Bestrahlungsrichtung der Beleuchtung (L) so eingestellt sind, dass sie nicht den gleichen Winkel in Bezug auf eine auf die Bodenfläche des Kulturgefäßes (1) senkrechten Achse aufweisen.

2. Zellbeobachtungsvorrichtung (2) nach Anspruch 1, **dadurch gekennzeichnet, dass** sie Folgendes umfasst:
eine Bewegungseinheit (6), die die Kamera (4) und die Beleuchtung (5) einstückig bewegt, während die Abbildungsrichtung der Kamera und die Bestrahlungsrichtung der Beleuchtung beibehalten werden, wobei
die Bewegungseinheit (6) den Fokus der Kamera auf die Bodenfläche jeder Stufe des gestapelten Kulturgefäßes einstellt, indem die Kamera (4) und die Beleuchtung (5) nach oben und nach unten bewegt werden.

3. Zellenbeobachtungsvorrichtung (2) nach einem der Ansprüche 1 und 2, **dadurch gekennzeichnet, dass** sie Folgendes umfasst:
eine Bewegungseinheit (6), die die Kamera (4) und die Beleuchtung (5) einstückig bewegt, während die Abbildungsrichtung der Kamera und die Bestrahlungsrichtung der Beleuchtung beibehalten werden, wobei
die Bewegungseinheit (6) den Bestrahlungsbereich des Inspektionslichts (L) und den Abbildungsbereich der Kamera (4) auf der Bodenfläche des Kulturgefäßes (1) verändert, indem die Kamera (4) und die Beleuchtung (5) lateral bewegt werden.

## Revendications

1. Appareil d'observation de cellules (2) comprenant une caméra (4) imageant des cellules contenues dans un récipient de culture (1) et un éclairage (5) irradiant les cellules avec une lumière d'inspection (L), **caractérisé en ce qu'**il comprend :
une chambre stérile (3), dont un intérieur est maintenu dans un état stérile et qui contient le récipient de culture (1) ; et une fenêtre d'observation transparente (11) prévue dans une partie inférieure de la chambre stérile (3) et présentant le récipient de culture placé au-dessus, dans lequel
la caméra (4) et l'éclairage (5) sont fournis au-dessous de la fenêtre d'observation, et les cellules dans le récipient de culture (1) sont imagées à travers la fenêtre d'observation (11) en réglant la mise au point de la caméra sur une surface inférieure du récipient de culture,
l'éclairage (5) émet la lumière d'inspection (L) sous la forme d'une lumière ponctuelle vers la surface inférieure du récipient de culture (1) en émettant de la lumière sous la forme d'un point où la lumière n'est pas diffusée, dans lequel une plage d'imagerie de la caméra (4) est positionnée à l'intérieur d'une plage d'irradiation de la lumière d'inspection (L) sur la surface inférieure du récipient de culture, et
l'appareil d'observation de cellules (2) est pourvu d'un mécanisme de réglage d'angle de telle sorte qu'une direction d'imagerie de la caméra (4) et une direction d'irradiation de l'éclairage (L) sont réglées de manière à ne pas être au même angle par rapport à un axe orthogonal à la surface inférieure du récipient de culture (1).

2. Appareil d'observation de cellules (2) selon la revendication 1, **caractérisé en ce qu'**il comprend :
une unité de déplacement (6) déplaçant la caméra (4) et l'éclairage (5) d'un seul tenant tout en maintenant la direction d'imagerie de la caméra et la direction d'irradiation de l'éclairage, dans lequel :
l'unité de déplacement (6) règle la mise au point de la caméra sur la surface inférieure de chaque étage des récipients de culture empilés en déplaçant la caméra (4) et l'éclairage (5) vers le haut et vers le bas.

3. Appareil d'observation de cellules (2) selon l'une quelconque des revendications 1 et 2, **caractérisé en ce qu'**il comprend
une unité de déplacement (6) déplaçant la caméra (4) et l'éclairage (5) d'un seul tenant tout en maintenant la direction d'imagerie de la caméra et la direction d'irradiation de l'éclairage, dans lequel :
l'unité mobile (6) modifie la plage d'irradiation de la lumière d'inspection (L) et la plage d'imagerie de la caméra (4) sur la surface inférieure du récipient de culture (1) en déplaçant latéralement la caméra (4) et l'éclairage (5).
